# EUROPEAN PATENT SPECIFICATION

(11) **EP 1 594 399 B1**
(45) Date of publication and mention of the grant of the patent: **29.11.2006**
(21) Application number: 04712611.5
(22) Date of filing: 19.02.2004
(51) Int. Cl.: A61B 5/026, A61B 5/00

(54) **TISSUE ASSESSMENT**
GEWEBEBEURTEILUNG
EVALUATION DE TISSUS

(30) Priority: 19.02.2003 GB 0303797
(43) Date of publication of application: 16.11.2005
(73) Proprietor: HUNTLEIGH TECHNOLOGY PLC, Luton, Bedfordshire LU1 1TD (GB)
(72) Inventor: GOUGH, Nigel, Cardiff CF14 6HD (GB)
(74) Representative: Thaker, Shalini
(86) International application number: PCT/GB2004/000674
(87) International publication number: WO 2004/073514

(56) References cited:
- US-A- 4 538 618
- US-B1- 6 178 342

## Description

The present invention relates to a tissue assessment device and method. More particularly, the present invention relates to a device and method for the assessment of microvasculature damage in the tissue. The invention is applicable to conditions that affect the haemodynamics of the microvasculature such as ischaemia, early stage pressure damage, white finger syndrome (WFS), Reynaud's syndrome and diabetic peripheral neuropathy.

Skin evaluation devices are known that use reflectance spectroscopy to assess skin perfusion. A known device uses a sliding blanch technique to push blood out of the skin immediately below an optical sensor and a photo detector. The optical sensor delivers light to the skin and measures the intensity of light reflected back. The sliding blanch technique involves the operator holding one end of a probe against the skin and either pushing or pulling it along the surface of the skin a ridge on the probe causing the blanching. Unfortunately, it has been found that this device is acceptable only with a skilled practitioner thereby restricting its use

US 6 178 342 discloses a tissue assessment device according to the preamble of claim 1.

An object of the present invention is to seek improvements.

According to the present invention, there is provided a tissue microvasculature assessment device as claimed in claim 1. The information provided by the device is input into a classification algorithm trained using reference data and analysed to provide discrimination of microvasculature damage.

The application of pressure by the inflated diaphragm is gentle onto the tissue surface and does not cause pain or discomfort. Preferably, the application of pressure on the tissue surface is constant for a predetermined time. Alternatively, the pressure applied can be pulsed and/or of varying pressure. More preferably, the pressure applied is rapidly released to allow for blood reflow into the area.

Preferably, the diaphragm is transparent so that light transmission and return paths can pass through the diaphragm.

Preferably, when the application of pressure is released the diaphragm relaxes and moulds itself to the contour of the tissue surface preventing extraneous light from entering the optical sensor and reducing measurement errors. Also provided, is a diaphragm for use with such a tissue microvasculature assessment device, the diaphragm comprising a transparent flexible membrane adapted to be fitted over the end of the tissue microvasculature assessment device.

Preferably the values of returned light can be analysed to provide assessment of limb ischaemia and assessment of diabetic peripheral neuropathy.

An embodiment of the present invention is described below, by way of example only, with reference to the accompanying drawings in which:
Figure 1 is a system diagram of a tissue microvasculature assessment device according to the invention;
Figure 2 is a schematic diagram of a preferred embodiment of the tissue microvasculature assessment device;
Figure 3 shows the optical system of the device in Figure 2; and
Figure 4 shows the one set of results obtained from the device.

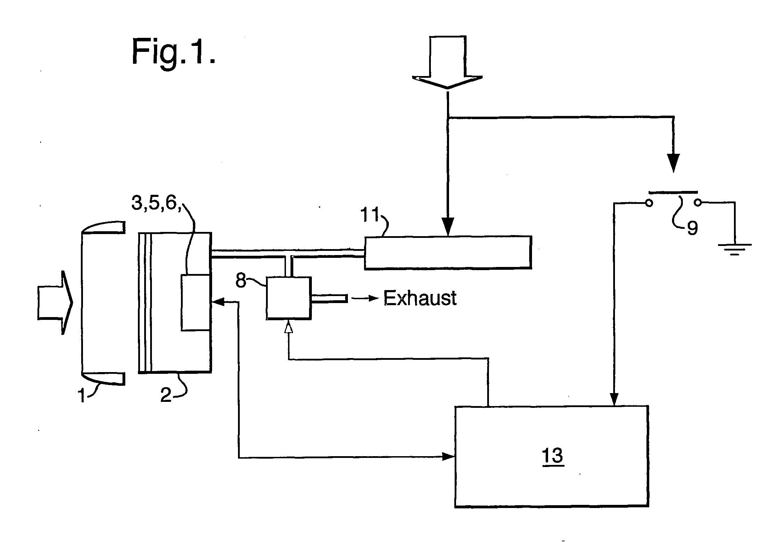

Referring to Figure 1, the tissue microvasculature assessment system consists of a fluid pressure system 1,2,11 to produce a blanch, an actuator 12 to actuate the blanch, an optical system to illuminate the blanch and receive the reflected signals and a micro-controller 13 to control the blanch process, the optical process and take measurements.

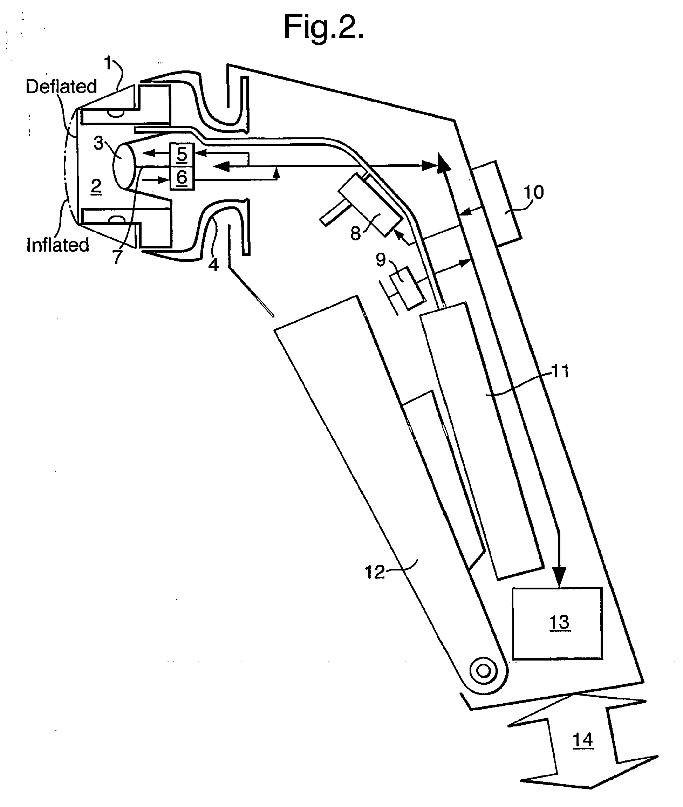

As shown in Figure 2, the preferred embodiment has a bladder 11 with stiff walls constituting a pump, an open ended chamber 2 housing the optical system 3, 5, 6 and a transparent, flexible diaphragm 1 stretched over the open end of chamber 2, all comprising the fluid pressure system to apply a blanch force to the tissue surface. The diaphragm 1 can also be inflated by any other suitable means, such as a small pump activated by a switch. The switch could be optical or mechanical or similar.

The diaphragm 1 is fitted over the end of the chamber 2 thereby sealing the chamber 2 and causing the whole fluid pressure system to be sealed. Compression of bladder 11 by actuator 12 displaces the fluid within the bladder 11 into chamber 2 causing diaphragm 1 to inflate to initiate the blanch onto the tissue surface. To remove the applied pressure, the diaphragm 1 is deflated either by releasing the actuator 12 or opening an exhaust valve 8. The exhaust valve 8 serves to balance the internal pressure with the external atmospheric pressure before making a measurement, and to rapidly deflate the diaphragm 1 after a timed blanch force has been applied before making the refill measurement.

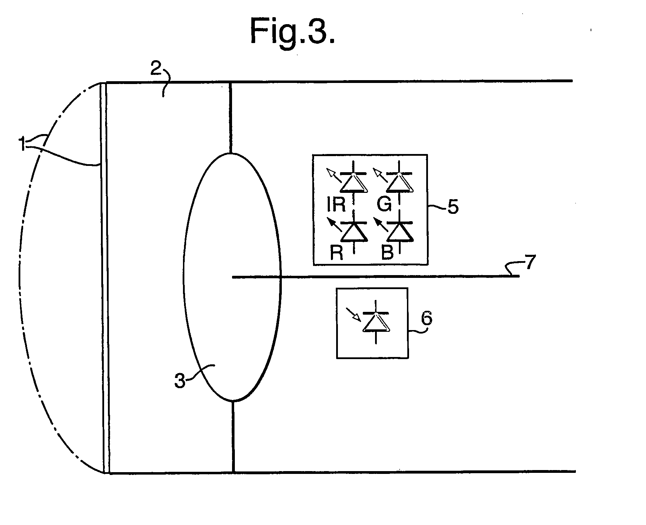

The micro-controller 13 communicates with the external systems, for example a display, controls the analogue to digital conversion of the received reflectance signal from the optical sensor 3,5,6; controls the light sources in the optical sensor 3,5,6; and controls the exhaust valve 8. The optical sensor comprises a light source 5 including infrared, red, green and blue LEDs and a photo detector 6. The optical system typically, but not exclusively, consists of several LEDs the wavelengths of which are chosen to suit the application. A typical combination may be infrared, red, green and blue. For the measurement of returned light a photo-detector 6 is used. The light to and from these components, may be coupled to the tissue surface by a fibre optic bundle or directly through a lens 3 (see Figure 3). The light source 5 and the photo-detector 6 are separated by a baffle 7 to prevent light from the light source 5 coupling directly with the photo-detector 6. The diaphragm 1 is of translucent silicon allowing light to pass through to the target tissue surface area.

In use, diaphragm 1 is attached to the probe head 2. and the face of the diaphragm 1 held against the tissue surface. A switch 10 initiates the calibration process. When calibration is complete, an audible or visual prompt informs the operator to squeeze the actuator 12. Actuator 12 compresses bladder 11 to displace the fluid, in this case air, within the bladder 11 into chamber 2 causing diaphragm 1 to inflate thus applying a force to the tissue surface to initiate a blanch. The blanch is timed by micro-controller 13, and after a predetermined time, the exhaust valve 8 is opened to rapidly deflate the diaphragm 1 to remove the blanching force. Throughout the blanching, the optical system 3,5,6 illuminates the blanch area and the returned light data is collected at regular intervals for each wavelength from the point when the blanch is initiated, throughout the blanching and a predetermined time thereafter. Although the preferred embodiment shows the arrangement as shown in Figure 2, other arrangements of the invention are possible in order to provide the tissue assessment device.

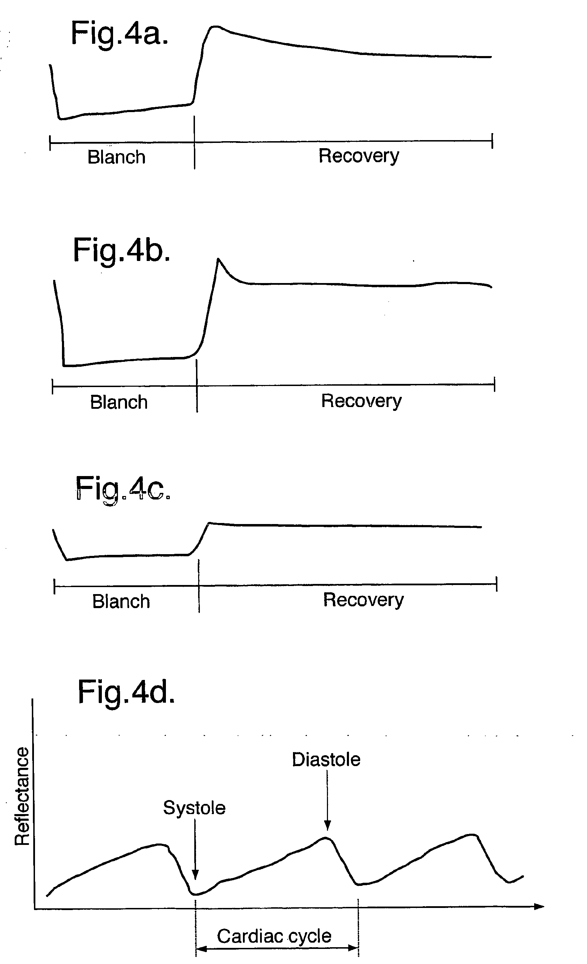

Typical blanch signals are shown in Figure 4. Figure 4a shows a typical normal signal with a slow refill curve during the recovery period. Figure 4b shows a refill curve with fast refill curve during the recovery phase. Figure 4c shows the curve typical of a nonblanching erythema, that is when the tissue microvasculature is damaged and there is no dynamic recovery curve since none of the blood has been blanched from the target tissue area. Furthermore, the device detects microvasculature pulsation (Figure 4d) associated with inflammation and hyperaemic response in those areas of tissue which, unlike the soles of the feet and the palms of the hands, are not normally densely vascularised and therefore where a pulse is normally not seen. This pulsation in conjunction with the dynamic refill signal provides a powerful classification data set to train a classification algorithm.

Such classification algorithms include Artificial Neural Networks (ANN) or polynomial curve fitting algorithms. The output of these classification algorithms can be communicated to the operator either by a simple display in the hand unit or on a host computer linked by the bi-directional data link 14. For example, in the case of pressure area tissue damage the information relayed to the operator is in the form of 3 categories, 1 NORMAL (non red area blanchable with normal refill curve), 2 BLANCHING HYPERAEMIA (red area which may not be seen in pigmented skin, that is blanchable with a shorter refill curve), 3 NONBLANCHING HYPERAEMIA (persistent red area which may not be seen in pigmented skin, that does not show a visible blanch.) Also, any blood supply manifest by a pulsatile component implying a resolvable condition can also be presented on the screen to the operator. Absence of such a pulsatile component could indicate severe damage.

Alternatively, where the assessment device is used for the assessment of critical limb ischaemia, the output takes the form of 2 categories such as, 1 SUFFICIENT FLOW, 2 INSUFFICIENT FLOW.

Further, it is also possible to analyse the data collected to determine physiologically related indices, for example, calculating the polynomial coefficients of curves fitted to the refill data or ratios of systolic and diastolic points in the pulsatile waveform or gradiants of the pre and post systolic peaks/troughs pulse waveform to provide an indication of tissue state.

Tissue assessment using the device according to the invention is easy to use without requiring any particular training while at the same time enhancing measurement repeatability. The invention provides a simple low skill tissue assessment device that is highly reliable. The device simulates the method used by Tissue Viability Specialists blanching the skin with the tip of the finger but provides a consistent blanch and a repeatable, measurable diagnosis regardless of patient skin colour.

Moreover, the measurements are taken from the same area of tissue that the device is calibrated on with the area of measurement larger than previous arrangements thereby giving stronger signals and the pressure applied for blanching is repeatable. The term tissue used throughout the description covers skin and any other tissues including internal body membranes and tissue surfaces exposed by surgical means, where such assessment is beneficial.

## Claims

1. A tissue assessment device comprising an optical system (5, 6, 7) to deliver and collect light to and from a tissue surface area, and a means to apply fluid pressure (1, 2, 11) onto a tissue surface area to initiate a blanch to skin immediately below the optical sensor and removed thereafter, **characterised in that** the means of applying fluid pressure is a diaphragm (1) comprising a transparent flexible membrane adapted to be fitted over the end of the tissue assessment device, inflated by air to apply pressure onto the tissue surface and deflated to release the applied pressure, and the optical system (3, 5, 6) comprises a light source (5) of different wavelengths, the light source (5) illuminating the blanch area and the return light data collected at regular intervals for each wavelength from the point when the blanch is initiated, throughout the blanching and a predetermined time after, the data providing a variation with time of blood reflow into the tissue area previously blanched.

2. A tissue assessment device as claimed in claim 1 **characterised in that** varying pressure is applied onto the area.

3. A tissue assessment device as claimed in claim 3 **characterised in that** the diaphragm (1) relaxes and moulds itself to the contour of the tissue when the pressure is released preventing extraneous light from entering the optical sensor (6) and reducing measurement errors.

4. A tissue assessment device as claimed in any preceding claim **characterised in that** the values of returned light are analysed to provide assessment of limb ischaemia.

5. A tissue assessment device as claimed in any preceding claim **characterised in that** the values of returned light are analysed to provide assessment of diabetic peripheral neuropathy.

## Patentansprüche

1. Vorrichtung zur Gewebebeurteilung mit einem optischen System (5, 6, 7), um Licht zu einem Gewebeoberflächenbereich zu liefern und von diesem Licht zu sammeln, und einer Einrichtung zum Aufbringen von Fluiddruck (1, 2, 11) auf einen Gewebeoberflächenbereich, um eine Bleichung der Haut unmittelbar unter dem optischen Sensor auszulösen und danach zu entfernen, **dadurch gekennzeichnet, dass** die Einrichtung zum Aufbringen von Fluiddruck ein Diaphragma (1) ist, mit einer transparenten, flexiblen Membran, die so ausgebildet ist, dass sie über dem Ende der Gewebebeurteilungsvorrichtung befestigt ist, die durch Luft aufgeblasen wird, um Druck auf die Gewebeoberfläche auszuüben und abgelassen wird, um den ausgeübten Druck zu lösen, und dass das optische System (3, 5, 6) eine Lichtquelle (5) mit unterschiedlichen Wellenlängen aufweist, die Lichtquelle (5) die gebleichte Fläche beleuchtet und die zurückgekehrten Lichtdaten, welche in regelmäßigen Intervallen für jede Wellenlänge von dem Punkt an, zu welchem das Bleichen ausgelöst wurde, während des Bleichens bis zu einer vorbestimmten Zeit danach gesammelt wurden, wobei die Daten eine Änderung mit der Zeit des Blutrückflusses in dem Gewebebereich, der zuvor gebleicht worden ist, bereitstellen.

2. Vorrichtung zur Gewebebeurteilung nach Anspruch 1, **dadurch gekennzeichnet, dass** der Bereich mit einem variierenden Druck beaufschlagt wird.

3. Vorrichtung zur Gewebebeurteilung nach Anspruch 3, **dadurch gekennzeichnet, dass** das Diaphragma (1) entspannt und sich selbst an die Kontur des Gewebes bei Lösen des Druckes anschmiegt, um zu verhindert, dass Licht von außen in den optischen Sensor (6) eintritt und Messfehler verhindert.

4. Vorrichtung zur Gewebebeurteilung nach einem der vorstehenden Ansprüche, **dadurch gekennzeichnet, dass** die Werte des zurückgekehrten Lichtes analysiert werden, um eine Beurteilung der Ischämie der Extremität bereitzustellen.

5. Vorrichtung zur Gewebebeurteilung nach einem der vorstehenden Ansprüche, **dadurch gekennzeichnet, dass** die Werte des zurückgekehrten Lichtes analysiert werden, um eine Beurteilung der peripheren Diabetiker-Neuropathie bereitzustellen.

## Revendications

1. Dispositif d'évaluation de tissus comprenant un système optique (5, 6, 7) pour délivrer de la lumière à une zone de surface de tissu et collecter de la lumière provenant de cette dernière, et un moyen pour appliquer une pression de fluide (1, 2, 11) sur une zone de surface de tissu pour établir un blanc sur la peau juste en dessous du capteur optique et la supprimer ensuite, **caractérisé en ce que** le moyen d'application de pression de fluide est un diaphragme (1) comprenant une membrane souple transparente adaptée pour être ajustée sur l'extrémité du dispositif d'évaluation de tissus, gonflé d'air pour appliquer une pression sur la surface de tissu et dégonflé pour relâcher la pression appliquée, et le système optique (3, 5, 6) comprend une source de lumière (5) de différentes longueurs d'onde, la source de lumière (5) illuminant la zone blanche et les données de lumière de renvoi collectées à intervalles réguliers pour chaque longueur d'onde depuis le moment où le blanc a été établi, pendant le blanchiment et un temps prédéterminé après, les données fournissant une variation avec le temps du reflux sanguin dans la zone de tissu auparavant blanchie.

2. Dispositif d'évaluation de tissus selon la revendication 1, **caractérisé en ce qu'**une pression variable est appliquée à la zone.

3. Dispositif d'évaluation de tissus selon la revendication 3, **caractérisé en ce que** le diaphragme (1) se détend et se moule lui-même sur le contour du tissu lorsque la pression est relâchée, empêchant la lumière extérieure d'entrer dans le capteur optique (6) et réduisant les erreurs de mesure.

4. Dispositif d'évaluation de tissus selon l'une quelconque des revendications précédentes, **caractérisé en ce que** les valeurs de lumière renvoyée sont analysées pour fournir une évaluation d'ischémie du membre.

5. Dispositif d'évaluation de tissus selon l'une quelconque des revendications précédentes, **caractérisé en ce que** les valeurs de lumière renvoyée sont analysées pour fournir une évaluation de neuropathie périphérique diabétique.
